## Europäisches Patentamt

## European Patent Office

## Office européen des brevets

(19)

(11) Publication number: **0 107 383**
**B1**

(12)

# EUROPEAN PATENT SPECIFICATION

(45) Date of publication of patent specification: **06.08.86**

(51) Int. Cl.⁴: **C 12 Q 1/56,** G 01 N 33/50

(21) Application number: **83305817.5**

(22) Date of filing: **28.09.83**

(54) **Diagnostic activated partial thromboplastin reagent.**

(30) Priority: **29.09.82 US 427023**

(43) Date of publication of application:
**02.05.84 Bulletin 84/18**

(45) Publication of the grant of the patent:
**06.08.86 Bulletin 86/32**

(84) Designated Contracting States:
**AT BE CH DE FR GB IT LI LU NL SE**

(56) References cited:
**EP-A-0 020 895
DE-B-2 316 430
US-A-3 183 159**

**AMERICAN JOURNAL OF CLINICAL
PATHOLOGY, vol. 62, December 1974, A.L.
BABSON, S.R. BABSON "Comparative
Evaluation of a Partial Thromboplastin
Reagent Containing a Non-settling Particulate
Activator" pages 856-860**

**JOURNAL OF CLINICAL PATHOLOGY, vol. IX,
1958 J. MARGOLIS "The Kaolin Clotting Time"
pages 406-409**

(73) Proprietor: **ORTHO DIAGNOSTIC SYSTEMS
INC.**
**One Johnson & Johnson Plaza
New Brunswick New Jersey 08933 (US)**

(72) Inventor: **Hughes, James Earl
110 Fernwood Drive Box No. 462
Tannersville, PA 18372 (US)**
Inventor: **Page, Eileen
144 Belt Road
Phillipsburg New Jersey 08865 (US)**
Inventor: **Semar, Martin
77 Franklin Street
Tenafly New Jersey 07670 (US)**

(74) Representative: **Jones, Alan John et al
CARPMAELS & RANSFORD 43 Bloomsbury
Square
London, WC1A 2RA (GB)**

# 0 107 383

## Description

The present invention relates to the field of diagnostic coagulation testing and more specifically this invention provides a partial thromboplastin reagent with a nonsettling surface contact Factor XII activator.

### Background of the Invention

Hemostasis is a complicated life saving procedure involving blood vessels, plasma, platelets, vessel walls, neural and humoral activity, all for the purpose of stanching bleeding. Although there has been much research, especially within the last few decades, concerning the process of coagulation specifically and hemostasis in general, there is much that is not completely understood particularly with regard to the specific details concerning the interaction of contributing mechanisms. For instance, it is now known that there are no less than 35 compounds which take part in the formation of a firm blood clot. Ostensibly, blood coagulation is thus one of the more complicated chemical processes occurring within the body. In fact, there are so many separate components within the scheme of coagulation that the International Committee on Nomenclature for Blood Coagulation formed by the International Hematology Congress has adopted a series of names which have now come into general acceptance. The most common of these names (with generally employed alternatives) and factor designations include the following:

| | |
|---|---|
| Factor I | Fibrinogen |
| Factor II | Prothrombin |
| Factor III | Thromboplastin |
| Factor IV | Calcium |
| Factor V | Proaccelerin, labile factor |
| Factor VI | (no longer used) |
| Factor VII | Serum prothrombin conversion accelerator (SPCA), stable factor |
| Factor VIII | Antihemophilic factor (AHF) |
| Factor IX | Christmas factor, plasma thromboplastin component (PTC) |
| Factor X | Stuart Factor, Stuart-Prower factor |
| Factor XI | Plasma thromboplastin antecedent (PTA) |
| Factor XII | Hageman factor |
| Factor XIII | Fibrin stabilizing factor |
| Profibrinolysin | Plasminogen |
| Fibrinolysin | Plasmin |

Diagnostic determination of the presence and quantitation of various of the above factors is important as many clinically relevant diseases associated with abnormal clotting are associated therewith. For instance, the absence of Factor VIII is the most frequent and serious cause of genetically determined clotting defects such as hemophilia A which has been recognized for over 2,000 years. A deficiency in plasma concentration of Factor IX is generally associated with the genetically determined defect responsible for hemophilia B. Hemorrhagic disease results from deficiencies in Factors X and XI whereas deficiencies in Factor XII induce longer clotting times but still permit eventual clotting. Diseases of the liver or Vitamin K deficiencies have also been associated with abnormal levels of clotting Factors II, VII, IX and X as these are predominantly produced by the liver. Other causes of abnormal clotting may be related to reduced platelet counts, thrombocytopenia, often associated with pernicious anemia, certain drug therapies, irradiation or increased peripheral destruction by antibodies.

The clinical value of testing for coagulation times is not limited to the detection of genetic or pathological disease states but is also useful in the regulation of anticoagulant therapy. Anticoagulants typically inhibit the coagulation mechanism such as by the heparin mediated inhibiton of Factor X by Antithrombin III. Deficiencies in the clotting mechanisms due to congenital defects, pathological conditions, or anticoagulation therapy have been discussed generally in Chapter 7, "Coagulation and Hemostasis" by Robert D. Langdell, in Clinical Diagnosis, Davidson and Henry, 1979.

As commonly understood, coagulation may occur by two pathways, the so called intrinsic pathway and extrinsic pathway. The former is generally triggered by the presence of a surface (thought to activate Factor XII) and, with the presence of phospholipids and calcium, through a number of steps eventually stimulates the formation of a stabilized fibrin clot. The partial thromboplastin time (PTT) test typically measures the intrinsic pathway wherein most congenital deficiencies occur. Consequently this type of test serves as an excellent persurgical coagulation screening test; however, since the reagent employed in this test is typically a platelet substitute, the PTT test does not measure platelet activity.

The extrinsic pathway is generally triggered by injury to tissue and the resultant exudation of tissue thromboplastin acts on Factors VII and X which, following a series of steps, thereafter forms a fibrin clot. The extrinsic pathway is generally tested by the so called one-stage prothrombin time test to thereby detect most of the factors depressed by oral anticoagulant drugs for anticoagulant therapy control. The thrombin time test typically measures the quantity and reactivity of fibrinogen. This test is a rapid, semiquantitative test and is consequently the test of choice for intravascular clotting and fibrinolysis analysis. The above is presented in review in accompanying Table I as well as a booklet entitled "Concentric Concepts of Coagulation 1971, 1975" available from Ortho Diagnostics, Inc., Raritan, New Jersey.

## TABLE 1

INTRINSIC PATHWAY

TRIGGER: SURFACE

EXTRINSIC PATHWAY

TRIGGER: TISSUE INJURY

Platelets

III Tissue Thromboplastin

XII   + Phospholipids

[Partial
Thromboplastin
Time (PTT)]

XI   + Ca

VII  (One-stage Prothombin Time)

IX

VIII

X

Fibrinogen

V ⟶ II ⟶ Thrombin ⟶ I ⟶ Fibrin ⟶ XIII ⟶ Stabilized
                                    Clot              Fibrin Clot

Prothrombin

(Thrombin Time Test)

Roman Numerals indicate Factor designations.

Perhaps because of its apparently simpler nature in connection with more readily discernible initiation causes i.e., injury, the mechanisms involved in the extrinsic pathway were more suscepible to understanding than those involved with the intrinsic pathway. For instance, as late as 1957, in an article by J. Margolis entitled "Initiation of Blood Coagulation by Glass and Related Surfaces" appearing in the Journal of Physiology, 137,95—109 (1957), it was clear that there was still much to be learned concerning the "in vitro" mechanisms. It was observed that the contact of various surfaces with plasma including platelet poor plasma resulted in plasma clotting. The Margolis study reported findings regarding clotting time versus platelets per cubic millimeter versus contact surface and attempted to draw conclusions therefrom. It was noted that by providing an artificial surface such as glass, shortened clotting times could be observed. Other particulate activators have been employed in addition to glass and these include colloidal silica, celite, kaolin, and ellagic acid. Reagents containing these particular activators have been described in an article by Babson et al., Comparative Evaluation of a Partial Thromboplastin Reagent Containing a Nonsettling, Particulate Activator A.J.C.P. 62, 856—860 (December 1974). That article stressed the greater sensitivity involved with the use of colloidal silica. Further information regarding the use of kaolin as an activator may be obtained by reference to Margolis, "The Kaolin Clotting Time", J. Clin. Path. 11,406—409 (1959). Still another diagnostic reagent (actually a PTT reagent) has employed cholanic acid and a water soluble heavy metal salt and is described in U.S. Patent No. 3,983,004 to Trobiesh et al. Still another reagent, described by Lena Han et al. in U.S. Patent No. 3,395,210, utilizes bentonite and diatomaceous earth particles.

These reagents, employing activators for reducing the clotting time of normal plasma, have generally suffered from inherent defects making it necessary for technicians to exercise great care in order to obtain reproducible results. For instance, those agents employing siliceous materials require thorough agitation prior to each use in order to avoid the otherwise rapid settling of the silica from the saline suspension. Further, these tests tend not to lend themselves to automated optical systems due to the opacity of such suspensions. See for explanation and review, Canadian Patent No. 812,215 to Baker et al. That patent attempted to avoid the problems associated with particulate activators by employing ellagic acid. Such a product, Activated THROMBOFAX™ Reagent-Optimized is presently available from Ortho Diagnostic Systems Inc., Raritan, New Jersey and is further described in U.S. Patent No. 3,486,981 to Speck. Still further explanation may be had regarding the activation of Factor XII by solutions containing ellagic acid by reference to Journal of Laboratory and Clinical Medicine, Volume 63, No. 3:359—375 (March 1964) entitled "Activation of Hageman Factor by Solutions of Ellagic Acid" by Ratnoff et al.

While the Ortho Activated THROMBOFAX™ Reagent in its optimized state works well, it is an object of the present invention to provide a silica activated partial thromboplastin reagent having superior sensitivity to heparinized plasma in the therapeutic range and to factor deficient plasmas.

Diagnostic reagents employing fumed silica as an activator are not new. See, for instance, U.S. Patent No. 3,880,714 to Babson as well the product package insert for Automated APTT available from General Diagnostics. The Babson invention was claimed to represent an advance over prior silica particles due to the elimination of constant stirring in order to maintain suspension of the particles. It should, however, be carefully noted that the Babson reagent employs a *fumed* silica particle as the critical activator substance. It is an object of the present invention to also obviate the need of any type of resuspending activities without relying on fumed silica particles.

Studies attempting to explain the mechanism of silica stimulated activation as well as comparisons of silica effectiveness on weight basis have been described by Margolis, Some Physical Aspects of Plasma/Surface Reactions in PROC. of Eighth International Congress of Hematology, Tokyo, Volume 3, 1962, Pages 672—673. Other studies relating to the effectiveness on coagulation of granules in general, including silica specifically, based on particle size are reported by Margolis in "The Effective Colloidal Silica on Blood Coagulation", Aust. J. Exp. Biol. 39:249—258 (1961).

DE—B2—2 316 430 to Behringwerke discloses a partial thromboplastin for use in a diagnostic reagent. The partial thromboplastin is extracted from human placentas and is preferably stabilised with a sugar alcohol or sodium glutaminate. The reagent contains the stabilised extract and kaolin.

US—A—3 183 159 to Singher discloses a partial thromboplastin time test reagent comprising ether solubilised cephalin stabilised by the presence of sodium ethylmercurithiosalicylate.

EP—A—0 020 895 to Behringwerke discloses a diagnostic partial thromboplastin reagent comprising a phospholipid extract derived from a plant such as soya bean, a water soluble Factor XII activator such as ellagic acid and a water soluble alkaline earth salt such as calcium chloride. The reagent is preferably buffered to a pH of about 7.4 by use of an amino acid such as glycine.

It has been discovered by the inventors hereof that the silica particles need not be fumed and in fact, it has been found preferable to use nonfumed silica particles in order to meet another object of the present invention, that of providing a reagent with an improved precision and optimal normal clotting time than those presently available.

Brief Summary of the Invention

The present invention provides a diagonstic partial thromboplastin reagent buffered to a pH from 7 to 8 comprising a phospholipid, an amino acid and a Factor XII activator, characterised in that: a) the phospholipid is a cephalin selected from soybean lecithins, brain tissue, nervous tissue, living organisms, rabbit cephalin and bovine cephalin, the cephalin being present in a final concentration from 0.25 to 10%; b) the buffer is HEPES, BES, BICINE, MOPS, TES or PIPES, and is present in a final concentration from 0.025M to 0.22M; c) the Factor XII activator is nonfumed colloidal silica having a particle diameter from 12 to 60 nm and a surface area from 50 to 230 $m^2/g$; and d) the amino acid is present in a final concentration from 1 to 10% as a suspending reagent.

The present invention provides reagents for diagnostically testing and evaluating the intrinsic coagulation pathway having superior shelf lifes. These reagents are generally optically clearer than those previously available thereby lending themselves to greater usefulness in instruments which measure optical density changes. As the reagents are preferably to be offered in liquid form, the avoidance of errors and contamination occurring with the reconstitution of lyophilized products is realized. Additionally, the reagents yield faster normal clotting times thereby providing increased precision as well as increased sensitivity to heparinized plasma.

As has been previously intimated, the exposure of human plasma to glass, kaolin, celite, or other negatively charged surfaces initiates contact activation which triggers the intrinsic coagulation pathway. When bound, the Hageman Factor (Factor XII) is 500 times more susceptible to cleavage by proteases than when solubilized within the plasma. Thus, it is presumed that the surfaces promote activation of the Hageman Factor indirectly by altering its structure thereby making it more susceptible to plasma proteases. Based on the above theory, the inventors believe, without wishing to be held to such belief, that the increased sensitivity to Hageman Factor deficiencies by the reagents of the instant invention may be traced to the provision of increased surface area by the employment of *nonfumed* colloidal silica. Such a nonfumed colloidal silica is available from DuPont under the name Ludox® and, it has been found preferable to employ the WP$^a$ grade of the Ludox® colloidal silica as well as from other sources.

The WP$^a$ Ludox® colloidal silica as described by DuPont, has a negative particle charge with an average diameter of 22 nm and a specific surface area of 140 square meters per gram. The particle is further characterized as $FIO_2$ 35 weight percent, pH 10.7 at 25C, 0.62 weight percent titratable alkali as $Na_2O$, 55 percent by weight $SiO_2$, 0.02 weight percent of chlorides, 0.05 weight percent of sulfates, and a viscosity of 4cP ($4.10^{-3}$ Pa.s). Pursuant to DuPont's brochure, this grade of colloidal silica as listed as a polishing agent for silicon wafers.

The DuPont material has ideally been employed as it comes from the reagent bottle, preferably in a concentration range of about 0.04—.4 percent with an optimal concentratioon being approximately 0.08 percent of the final formulation.

It has been preferable to include an activity enhancer such as manganous chloride in the range of approximately 0.2—4 percent of a 0.1 molar solution with the optimal range ideally in the range of about 1.2—2 percent of the final concentration. Again, without wishing to be held to theory, the present inventors believe that the double positive charge on the manganous ion acts to bring the cephalin and the silica in closer proximity causing the silica to be more effective in activating the Factor XII. Alternatively, other activity enhancers such as magnesium chloride, aluminum chloride, and barium chloride have been tested and found effective in substitution for manganous chloride. Other double or triply charged cations occurring across the same rows in the periodic table of elements such as copper chloride, nickel chloride, zinc chloride, and chromium chloride although untried, may be expected to be equally effective and advantageously employed in substitution.

In order to provide an optimal pH for maximizing effectiveness of the reagent, an organic buffer is employed such as the HEPES Buffer (N-2-Hydroxyethylpiperazine-N'-2-ethane suflonic acid). Ideally the HEPES Buffer will be employed in the final concentration range of approximately 0.025 M—0.22 M and a pH in the range of approximately 7 to 8. The preferred embodiment of the present invention employes HEPES Buffer having a final concentration of approximately 0.1 M and a pH of about 7.5 in order to effectuate optimal activation. Alternative embodiments may employ in substitution for the HEPES Buffer the following: BES Buffer (N,-N-bis-(2-hydroxyethyl)-2-aminoethane sulfonic acid); BICINE Buffer (N,N-bis-(2-Hydroxyethylglycine); MOPS Buffer (Morpholinopropane sulfonic acid); TES Buffer (N-Tris(hydroxymethyl)methyl-2-aminoethane sulfonic acid); PIPES Buffer (Piperazine-N,N'-bis(2-ethane sulfonic acid), all preferably adjusted in order to maximize stability at approximately a 50/50 equilibrium of disassociated-nondisassociated forms.

The plateletlike activity is provided preferably by a phospholipid such as rabbit or bovine brain cephalin extract, however, other sources such as plant tissues (for example, soybeam lecithin) and animal tissues (for example, lung and nervous tissue and highly vascularized organs) may also be advantageously employed in substitution. These phospholipids are typically ether acetone extacted in accordance with well-known techniques. Typically, the extract component comprises a 1.3 percent cephalin suspension mixed with 0.8 percent sodium chloride and preferably 1 to 10,000 parts of thimerosal as a preservative, however, other preservatives may be equally or more effective. The cephalin is ideally adjusted to have a final concentration (in the reagent) in the range of about 0.25—10 percent with the preferred optimal concentration as 2.0 percent of the final solution.

The reagent further comprises a suspending reagent for preventing the settling out of the activity enhancers. Such a suspending reagent is glycine which preferably provided in a final concentration in the range of about 4 to 5 percent. Also included within the reagent of the present invention will be distilled water or triple deionized water as appropriate in order to make up the volume to provide the above-specified concentration ranges.

A comparison of the clotting times exhibited among Ortho Activated THROMBOFAX™ (optimized), Generally Diagnostics Automated APTT (the fumed silica preparation), and the nonfumed colloidal silica and rabbit or bovine cephalin reagent of the present invention is presented in the accompanying Table II. That Table provides information concerning the clotting times of plasma having from 0 to 0.6 units of heparin per ml as well as the relative effectiveness with various deficiencies in Factors VIII, IX, X, XI, Fletcher and Ortho Abnormal Plasma Coagulation Control (APCC). To be noted is that although faster times are generally preferable than longer times, a time that is too fast (such as the 77 seconds exhibited by the Ortho Activated THROMBOFAX™ in 0.6 units heparinized plasma) provides a standard curve that is relatively flat and therefore felt by many to be difficult to interpret. Thus, the rabbit cephalin, nonfumed colloidal silica reagent of the present invention is the most preferred embodiment as it provides optimal clotting times which are neither too fast nor too slow thereby exhibiting superior sensitivity.

### TABLE 2

| | NORMAL PLASMA | HEPARINIZED PLASMA | | |
|---|---|---|---|---|
| | 0 Units/ml | 0.2 Units/ml | 0.4 Units/ml | 0.6 Units/ml |
| Activated THROMBOFAX™ (Optimized) | 24.8 | 40.2 | 55.25 | 77.0 |
| General Diagnostics Automated APTT | 28.85 | 58.5 | 85.45 | 133.5 |
| Non-fumed Silica using Rabbit Cephalin | 25.6 | 52.15 | 80.95 | 134.55 |
| Non-fumed Silica using Bovine Cephalin | 25.55 | -51.15 | 86.4 | 162.3 |

| | VIII | IX | X | XI | FLETCHER[1] | OAPCC[2] |
|---|---|---|---|---|---|---|
| Activated THROMBOFAX™ (Optimized) | 40.2 | 37.55 | 98.55 | 117.45 | 69.6 | 72.85 |
| General Disgnostics Automated APTT | 78.05 | 93.6 | 142.3 | 89.45 | 141.5 | 76.85 |
| Non-fumed Silica using Rabbit Cephalin | 59.9 | 64.55 | 100.5 | 85.0 | 115.4 | 74.95 |
| Non-fumed Silica using Bovine Cephalin | 61.05 | 65.4 | 103.35 | 89.05 | 133.8 | 75.05 |

VALUES LISTED ARE CLOTTING TIMES IN SECONDS

[1]Plasma Prekallikrein

[2]ORTHO Abnormal Plasma Coagulation Control available from Ortho Diagnostic Systems Inc.

A preferred reagent using nonfumed silica and rabbit cephalin comprises:

| | |
|---|---|
| rabbit cephalin | 2% |
| HEPES buffer | 0.1M |
| manganous chloride | 1.2 to 2% |
| glycine | 4 to 5% |
| nonfumed silica | 0.04 to 0.4% |
| water to 100% | |

**0 107 383**

A preferred reagent using nonfumed silica and bovine cephalin comprises:

| | |
|---|---|
| bovine cephalin | 2% |
| HEPES buffer | 0.1M |
| manganous chloride | 1.2 to 2% |
| glycine | 4 to 5% |
| nonfumed silica | 0.8% |
| water to 100% | |

Preferably, the reagents of the present invention are provided in liquid format in order to obviate contaminating and resuspending errors common with lyophilized products such as the fumed colloidal product of General Diagnostics.

**Claims**

1. A diagnostic partial thromboplastin reagent buffered to a pH from 7 to 8 comprising a phospholipid, an amino acid and a Factor XII activator, characterised in that:
   a) the phospholipid is a cephalin selected from soybean lecithins, brain tissue, nervous tissue, living organisms, rabbit cephalin and bovine cephalin, the cephalin being present in a final concentration from 0.25 to 10;
   b) the buffer is HEPES, BES, BICINE, MOPS, TES or PIPES, and is present in a final concentration from 0.025M to 0.22M;
   c) the Factor XII activator is nonfumed colloidal silica having a particle diameter from 12 to 60 nm and a surface area from 50 to 230 $m^2/g$; and
   d) the amino acid is present in a final concentration from 1 to 10% as a suspending reagent.
2. The reagent of claim 1, further comprising:
   e) manganous chloride, magnesium chloride, aluminium chloride, barium chloride, copper chloride, nickel chloride, zinc chloride, or chromium chloride as an activity enhancer in a final concentration from 0.2 to 4% of a 0.1M solution.
3. The reagent of claim 2, wherein the activity enhancer is present in a final concentration from 1.2 to 2.0% of a 0.1M solution.
4. The reagent of claim 2 or claim 3, wherein the activity enhancer is manganous chloride.
5. The reagent of any one of claims 1 to 4, wherein the cephalin is either extracted rabbit or bovine cephalin.
6. The reagent of claim 5, wherein the cephalin is present in a final concentration of approximately 2%.
7. The reagent of any one of claims 1 to 6, wherein the buffer is HEPES.
8. The reagent of claim 7, wherein the buffer is present in a final concentration of approximately 0.1M.
9. The reagent of any one of claims 1 to 8, wherein the nonfumed colloidal silica is present in a final concentration from 0.04 to 0.4%.
10. The reagent of claim 9, wherein the nonfumed colloidal silica is present in a final concentration of approximately 0.08%.
11. The reagent of any one of claims 1 to 10, wherein the amino acid suspending reagent is glycine.
12. The reagent of claim 11, wherein the glycine is present in a final concentration from 4 to 5%.
13. The reagent of any one of claims 1 to 12, further comprising water.
14. A method for the preparation of a reagent according to any one of claims 1 to 13, comprising mixing said ingredients in the stated amounts.

**Patentansprüche**

1. Diagnostisches aktiviertes Reagens auf partielles Thromboplastin, gepuffert auf einen pH-Wert von 7 bis 8 und mit einem Gehalt an einem Phospholipid, einer Aminosäure und Faktor XII-Aktivator, dadurch gekennzeichnet, daß:
   a) das Phospholipid ein unter Sojabohnenlecithinen, Hirngewebe, Nervengewebe, lebenden Organismen, Kaninchen-Cephalin und Rinder-Cephalin ausgewähltes Cephalin ist, wobei das Cephalin in einer Endkonzentration von 0,25 bis 10% vorlieght;
   b) der Puffer HEPES, DES BICINE, MOPS, TES oder PIPES ist und in einer Endkonzentration von 0,025 Mol bis 0,22 Mol vorliegt,
   c) der Faktor XII-Aktivator nicht-pyrogene kolloidale Kieselsäure mit einem Teilchendurchmesser von 12 bis 60 nm und einer Oberfläche von 50 bis 230 $m^2/g$ ist; und
   d) die Aminosäure in einer Endkonzentration von 1 bis 10% als ein Suspendiermittel zugegen ist.

8

2. Reagens nach Anspruch 1, mit einem zusätzlichen Gehalt an:

e) Mangan(II)chlorid, Magnesiumchlorid, Aluminiumchlorid, Bariumchlorid, Kupferchlorid, Nickelchlorid, Zinkchlorid oder Chromchlorid also Aktivitäts-Förderer in einer Endkonzentration von 0,2 bis 4% einer 0,1-molaren Lösung.

3. Reagens nach Anspruch 2, worin der Aktivitäts-Förderer in einer Endkonzentration von 1,2 bis 2,0% einer 0,1-molaren Lösung vorlieght.

4. Reagens nach Anspruch 2 oder 3, worin der Aktivitäts-Förderer Mangan(II)chlorid ist.

5. Reagens nach einem der Ansprüche 1 bis 4, worin das Cephalin entweder extrahiertes Kaninchen-Cephalin oder Rinder-Cephalin ist.

6. Reagens nach Anspruch 5, worin das Cephalin in einer Endkonzentration von annähernd 2% zugegen ist.

7. Reagens nach einem der Asprüche 1 bis 6, worin der Puffer HEPES ist.

8. Reagens nach Anspruch 7, worin der Puffer in einer Endkonzentration von annähernd 0,1 Mol vorlieght.

9. Reagens nach einem der Ansprüche 1 bis 8, worin die nichtpyrogene kolloidale Kieselsäure in einer Endkonzentration von 0,04 bis 0,4% vorliegt.

10. Reagens nach Anspruch 9, worin die nicht-pyrogene kolloidale Kieselsäure in einer Endkonzentration von annähernd 0,08% zugegen ist.

11. Reagens nach einem der Ansprüche 1 bis 10, worin das Aminosäure-suspendiermittel Glycin ist.

12. Reagens nach Anspruch 11, worin das Glycin in einer Endkonzentration von 4 bis 5% vorliegt.

13. Reagens nach einem der Ansprüche 1 bis 12, das zusätzlich Wasser enthält.

14. Verfahren zur Herstellung eines Reagens nach einem der Ansprüche 1 bis 13, umfassend das Vermischen der genannten Komponenten in den angegebenen Mengen.

## Revendications

1. Un réactif de diagnostic pour la détermination du temps de céphaline, tamponné à un pH de 7 à 8, comprenant un phospholipide, un amino-acide et un activateur du facteur XII, caractérisé en ce que:

a) le phospholipide est une céphaline choisie parmi les léchithines de soja, le tissu cérébral, le tissu nerveux, les organismes vivants, la céphaline de lapin et la céphaline bovine, la céphaline étant présente à une concentratioan finale de 0,25 à 10%;

b) le tampon est un tampon HEPES, BES, BICINE, MOPS, TES ou PIPES et est présent à une concentration finale de 0,025 M à 0,22 M;

c) l'activateur du facteur XII est une silice colloïdale non pyrogénée ayant un diamètre des particules de 12 à 60 nm et une surface spécifique de 50 à 230 m$^2$/g; et

d) l'amino-acide est présent à une concentration finale de 1 à 10% sous forme d'un réactif de suspension.

2. Le réactif de la revendication 1 comprenant de plus

e) du chlorure manganeux, de chlorure de magnésium, du chlorure d'aluminium, du chlorure de baryum, du chlorure de cuivre, du chlorure de nickel, du chlorure de zinc ou du chlorure de chrome comme stimulateur d'activaté à une concentration finale de 0,2 à 4% d'une solution 0,1 M.

4. Le réactif selon la revendication 2 ou la revendication 3 dans lequel le stimulateur d'activité est le chlorure manganeux.

5. Le réactif selon l'une quelconque des revendications 1 à 4 dans lequel le céphaline est soit de la céphaline extraite de lapin soit de la céphaline bovine.

6. Le réactif selon la revendication 5 dans lequel la céphaline est présente à une concentration finale d'environ 2%.

7. Le réactif selon l'une quelconque des revendications 1 à 6 dans lequel le tampon est l'HEPES.

8. Le réactif selon la revendication 7 dans lequel le tampon est présent à une concentration finale d'environ 0,1 M.

9. Le réactif selon l'une quelconque des revendications 1 à 8, dans lequel la silice colloïdale non pyrogénée est présente à une concentration finale de 0,04 à 0,4%

10. Le réactif selon la revendication 9 dans lequel la silice colloïdale non pyrogénée est présente à une concentration finale d'environ 0,08%.

11. Le réactif selon l'une quelconque des revendications 1 à 10 dans lequel l'amino-acide constituant le réactif de suspension est la glycine.

12. Le réactif selon la revendication 11 dans lequel la glycine est présente à une concentratioon finale de 4 à 5%.

13. Le réactif selon l'une quelconque des revendications 1 à 12 comprenant en outre de l'eau.

14. Un procédé pour la préparatioan d'un réactif selon l'une quelconque des revendications 1 à 13 comprenant le mélange desdits ingrédients dans les quantités indiquées.

9